# EUROPEAN PATENT APPLICATION

(11) **EP 2 497 499 A1**
(43) Date of publication of application: **12.09.2012**
(21) Application number: 11001999.9
(22) Date of filing: 10.03.2011
(51) Int. Cl.: A61K 47/48, A61P 35/00

(54) **Amatoxin-conjugates with improved linkages**

(71) Applicant: Heidelberg Pharma GmbH, 68526 Ladenburg (DE)
(72) Inventor: Simon, Werner, D-55595 Hüffelsheim (DE); Lutz, Christian, D-69469 Weinheim (DE); Müller, Christoph, D-69488 Birkenau (DE); Anderl, Jan, D-64397 Modautal (DE)
(74) Representative: Virnekäs, Bernhard

(57) **Abstract**

The invention relates to tumour therapy. In one aspect, the present invention relates to conjugates of an amatoxin and a target-binding moiety, e.g. an antibody, connected by certain linkages, which are useful in the treatment of cancer. In a further aspect the invention relates to pharmaceutical compositions comprising such conjugates.

## Description

### FIELD OF THE INVENTION

The invention relates to tumour therapy. In one aspect, the present invention relates to conjugates of an amatoxin and a target-binding moiety, e.g. an antibody, connected by certain linkages, which are useful in the treatment of cancer. In a further aspect the invention relates to pharmaceutical compositions comprising such conjugates.

### BACKGROUND OF THE INVENTION

Amatoxins are cyclic peptides composed of 8 amino acids. They can be isolated from *Amanita phalloides* mushrooms or prepared synthetically. Amatoxins specifically inhibit the DNA-dependent RNA polymerase II of mammalian cells, and thereby also the transcription and protein biosynthesis of the affected cells. Inhibition of transcription in a cell causes stop of growth and proliferation. Though not covalently bound, the complex between amanitin and RNA-polymerase II is very tight (K_{D} = 3 nM). Dissociation of amanitin from the enzyme is a very slow process, thus making recovery of an affected cell unlikely. When the inhibition of transcription lasts too long, the cell will undergo programmed cell death (apoptosis).

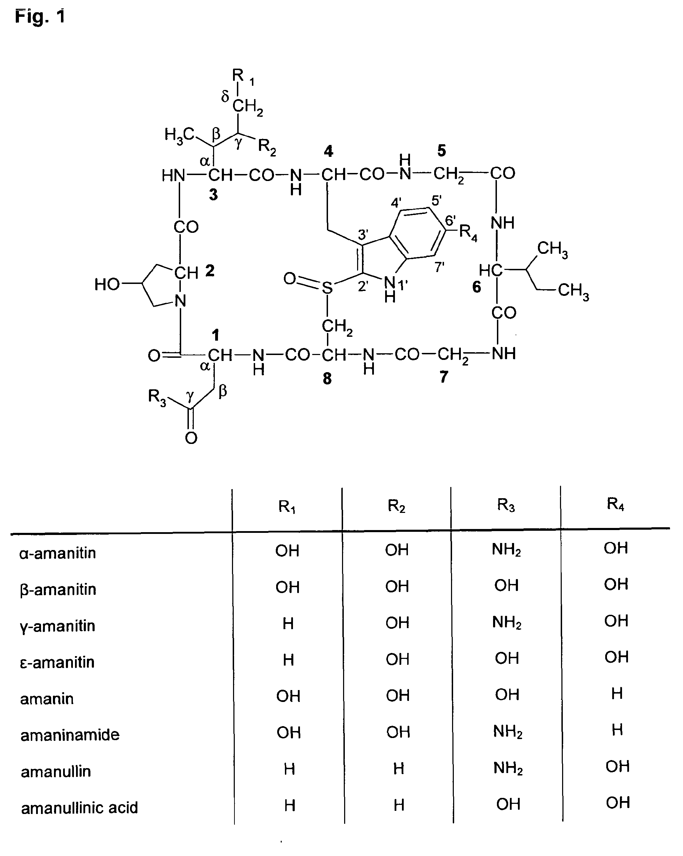

The use of amatoxins as cytotoxic moieties for tumour therapy had already been explored in 1981 by coupling an anti-Thy 1.2 antibody to α-amanitin using a linker attached to the indole ring of Trp (amino acid 4; see Figure 1) via diazotation (Davis & Preston, Science 1981, 213, 1385-1388). Davis & Preston identified the site of attachment as position 7'. Morris & Venton demonstrated as well that substitution at position 7' results in a derivative, which maintains cytotoxic activity (Morris & Venton, Int. J. Peptide Protein Res. 1983, 21 419-430).

Patent application EP 1 859 811 A1 (published November 28, 2007) described conjugates, in which the γ C-atom of amatoxin amino acid 1 of β-amanitin was directly coupled, i.e. without a linker structure, to albumin or to monoclonal antibody HEA125, OKT3, or PA-1. Furthermore, the inhibitory effect of these conjugates on the proliferation of breast cancer cells (MCF-7), Burkitt's lymphoma cells (Raji), and T-lymphoma cells (Jurkat) was shown. The use of linkers was suggested, including linkers comprising elements such as amide, ester, ether, thioether, disulfide, urea, thiourea, hydrocarbon moieties and the like, but no such constructs were actually shown, and no more details, such as attachment sites on the amatoxins, were provided.

Patent applications WO 2010/115629 and WO 2010/115630 (both published October 14, 2010) describe conjugates, where antibodies, such as anti-EpCAM antibodies such as humanized antibody huHEA125, are coupled to amatoxins via (i) the γ C-atom of amatoxin amino acid 1, (ii) the 6' C-atom of amatoxin amino acid 4, or (iii) via the δ C-atom of amatoxin amino acid 3, in each case either directly or via a linker between the antibody and the amatoxins. The suggested linkers comprise elements such as amide, ester, ether, thioether, disulfide, urea, thiourea, hydrocarbon moieties and the like. Furthermore, the inhibitory effects of these conjugates on the proliferation of breast cancer cells (cell line MCF-7), pancreatic carcinoma (cell line Capan-1), colon cancer (cell line Colo205), and cholangiocarcinoma (cell line OZ) were shown.

It is known that amatoxins are relatively non-toxic when coupled to large biomolecule carriers, such as antibody molecules, and that they exert their cytotoxic activity only after the biomolecule carrier is cleaved off. In light of the toxicity of amatoxins, particularly for liver cells, it is of outmost importance that amatoxin conjugates for targeted tumour therapy remain highly stable after administration in plasma, and that the release of the amatoxin occurs after internalization in the target cells. In this context, minor improvements of the conjugate stability may have drastic consequences for the therapeutic window and the safety of the amatoxin conjugates for therapeutic approaches.

### OBJECT OF THE INVENTION

Thus, there was a high need in the prior art for target-binding moiety amatoxin conjugates that are stable in plasma, so that harmful side effects to non-target cells are minimized.

### SUMMARY OF THE INVENTION

The present invention is based on the unexpected observation that targeting moieties can be attached to amatoxins via linkers at additional attachment sites on tryptophan amino acid 4, namely positions 1'-N without interference with the interaction of such amatoxins with their target, the DNA-dependent RNA polymerase II of mammalian cells.

Thus, the present invention relates to a conjugate comprising a target-binding moiety linked via a linker L to an amatoxin, or an analogue of an amatoxin, wherein the linker L is connected to the amatoxin via the 1'-N atom of amino acid 4.

In one aspect, the present invention relates to an amatoxin conjugate of Formula I 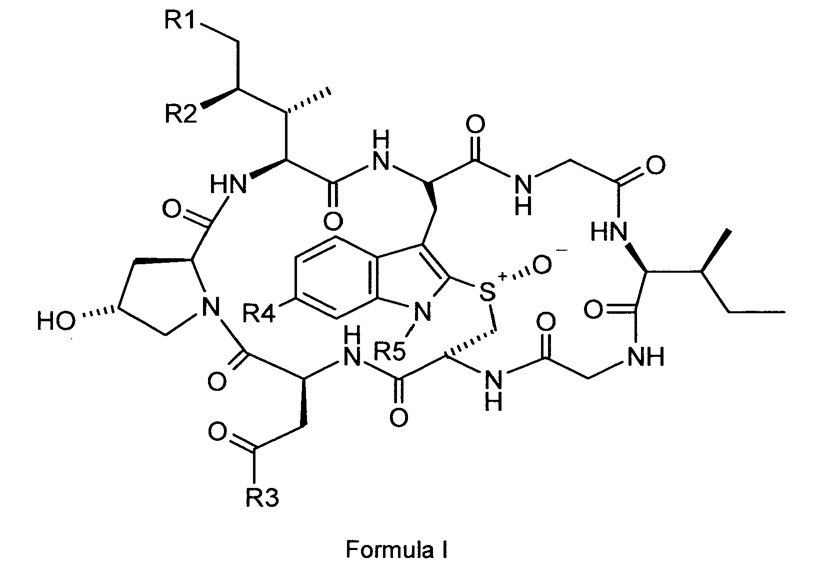 wherein:
- R1=: H or OH;
- R2=: H or OH;
- R3=: NH₂ or OH;
- R4=: OH or OC₁₋₆-alkyl; and
- R5=: L-T; wherein L is a linker; and T is a target binding moiety.

In a further aspect, the present invention relates to a pharmaceutical composition comprising the conjugate according to the present invention.

In another aspect, the present invention relates to an amatoxin-conjugation molecule of Formula I, wherein R1 to R4 are as defined above; and wherein R5 = L-X; wherein L is a linker; and X is a leaving group that can be replaced by a nucleophilic group of a target-binding moiety, particularly a primary amine.

In yet another aspect, the present invention relates to a method for synthesizing a conjugate of the present invention, comprising the step of reacting an amatoxin-conjugation molecule of the present invention with a target-binding moiety comprising a nucleophilic group, particularly a primary amine.

### BRIEF DESCRIPTION OF THE DRAWING

**Fig. 1** shows the structural formulae of different amatoxins. The numbers in bold type (1 to 8) designate the standard numbering of the eight amino acids forming the amatoxin. The standard designations of the atoms in amino acids 1, 3 and 4 are also shown (Greek letters α to γ, Greek letters α to δ, and numbers from 1' to 7', respectively).

**Fig. 2** shows the cytotoxic activity of Her-DSC-30.0378 [2.8] on a HER2-positive tumor cell line *in vitro* in a BrdU assay after incubation for 72 h.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step.

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety to the extent possible under the respective patent law. Nothing herein is to be construed as an admission that the invention is not entitled to antedate such disclosure by virtue of prior invention.

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Thus, the present invention relates to a conjugate comprising a target-binding moiety linked via a linker L to an amatoxin, or an analogue of an amatoxin, wherein the linker L is connected to the amatoxin via the 1'-N atom of amino acid 4.

In the context of the present invention, the term "conjugate" refers to a molecule comprising at least two different molecules linked by a covalent bond.

The term "target-binding moiety", as used herein, refers to any molecule or part of a molecule that can specifically bind to a target molecule or target epitope. Preferred target-binding moieties in the context of the present application are (i) antibodies or antigen-binding fragments thereof; (ii) antibody-like proteins; and (iii) nucleic acid aptamers. "Target-binding moieties" suitable for use in the present invention typically have a molecular mass of 40 000 Da (40 kDa) or more.

As used herein, a first compound (e.g. an antibody) is considered to "specifically bind" to a second compound (e.g. an antigen, such as a target protein), if it has a dissociation constant K_{D} to said second compound of 100 µM or less, preferably 50 µM or less, preferably 30 µM or less, preferably 20 µM or less, preferably 10 µM or less, preferably 5 µM or less, more preferably 1 µM or less, more preferably 900 nM or less, more preferably 800 nM or less, more preferably 700 nM or less, more preferably 600 nM or less, more preferably 500 nM or less, more preferably 400 nM or less, more preferably 300 nM or less, more preferably 200 nM or less, even more preferably 100 nM or less, even more preferably 90 nM or less, even more preferably 80 nM or less, even more preferably 70 nM or less, even more preferably 60 nM or less, even more preferably 50 nM or less, even more preferably 40 nM or less, even more preferably 30 nM or less, even more preferably 20 nM or less, and even more preferably 10 nM or less.

In the context of the present application the terms "target molecule" and "target epitope", respectively, refers to an antigen and an epitope of an antigen, respectively, that is specifically bound by a target-binding moiety. Preferably the target molecule is a tumour-associated antigen, in particular an antigen or an epitope which is present on the surface of one or more tumour cell types in an increased concentration and/or in a different steric configuration as compared to the surface of non-tumour cells Preferably, said antigen or epitope is present on the surface of one or more tumour cell types, but not on the surface of non-tumour cells. In particular embodiments, the target-binding moiety specifically binds to an epitope of HER-2/neu or epithelial cell adhesion molecule (EpCAM). In other embodiments, said antigen or epitope is preferentially expressed on cells involved in autoimmune diseases. In particular such embodiments, the target-binding moiety specifically binds to an epitope of the IL-6 receptor (IL-6R).

The term "antibody or antigen binding fragment thereof", as used herein, refers to immunoglobulin molecules and immunologically active portions of immunoglobulin molecules, i.e. molecules that contain an antigen-binding site that immunospecifically binds an antigen. Also comprised are immunoglobulin-like proteins that are selected through techniques including, for example, phage display to specifically bind to a target molecule, e.g. to the target protein Her-2/neu or EpCAM. The immunoglobulin molecules of the invention can be of any type (e.g., IgG, IgE, IgM, IgD, IgA and IgY), class (e.g., IgG1, IgG2, IgG3, IgG4, IgA1 and IgA2) or subclass of immunoglobulin molecule. "Antibodies and antigen-binding fragments thereof" suitable for use in the present invention include, but are not limited to, polyclonal, monoclonal, monovalent, bispecific, heteroconjugate, multispecific, human, humanized (in particular CDR-grafted), deimmunized, or chimeric antibodies, single chain antibodies (e.g. scFv), Fab fragments, F(ab')₂ fragments, fragments produced by a Fab expression library, diabodies or tetrabodies (Holliger P. et al., 1993), nanobodies, anti-idiotypic (anti-Id) antibodies (including, e.g., anti-Id antibodies to antibodies of the invention), and epitope-binding fragments of any of the above.

In some embodiments the antigen-binding fragments are human antigen-binding antibody fragments of the present invention and include, but are not limited to, Fab, Fab' and F(ab')₂, Fd, single-chain Fvs (scFv), single-chain antibodies, disulfide-linked Fvs (dsFv) and fragments comprising either a VL or VH domain. Antigen-binding antibody fragments, including single-chain antibodies, may comprise the variable domain(s) alone or in combination with the entirety or a portion of the following: hinge region, CL, CH1, CH2, and CH3 domains. Also included in the invention are antigen-binding fragments also comprising any combination of variable domain(s) with a hinge region, CL, CH1, CH2, and CH3 domains.

Antibodies usable in the invention may be from any animal origin including birds and mammals. Preferably, the antibodies are from human, rodent (e.g. mouse, rat, guinea pig, or rabbit), chicken, pig, sheep, goat, camel, cow, horse, donkey, cat, or dog origin. It is particularly preferred that the antibodies are of human or murine origin. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins, as described for example in U.S. Patent No. 5,939,598 by Kucherlapati & Jakobovits.

The term "antibody-like protein" refers to a protein that has been engineered (e.g. by mutagenesis of loops) to specifically bind to a target molecule. Typically, such an antibody-like protein comprises at least one variable peptide loop attached at both ends to a protein scaffold. This double structural constraint greatly increases the binding affinity of the antibody-like protein to levels comparable to that of an antibody. The length of the variable peptide loop typically consists of 10 to 20 amino acids. The scaffold protein may be any protein having good solubility properties. Preferably, the scaffold protein is a small globular protein. Antibody-like proteins include without limitation affibodies, anticalins, and designed ankyrin repeat proteins (for review see: Binz et al. 2005). Antibody-like proteins can be derived from large libraries of mutants, e.g. be panned from large phage display libraries and can be isolated in analogy to regular antibodies. Also, antibody-like binding proteins can be obtained by combinatorial mutagenesis of surface-exposed residues in globular proteins.

The term "nucleic acid aptamer" refers to a nucleic acid molecule that has been engineered through repeated rounds of *in vitro* selection or SELEX (systematic evolution of ligands by exponential enrichment) to bind to a target molecule (for a review see: Brody and Gold, 2000). The nucleic acid aptamer may be a DNA or RNA molecule. The aptamers may contain modifications, e.g. modified nucleotides such as 2'-fluorine-substituted pyrimidines.

As used herein, an "analogue" of a compound is structurally related but not identical to the compound and exhibits at least one activity of the compound. The compound to which the analogue is compared is known as the "parent" compound. The afore-mentioned activities include, without limitation: binding activity to another compound; inhibitory activity, e.g. enzyme inhibitory activity; toxic effects; activating activity, e.g. enzyme-activating activity. It is not required that the analogue exhibits such an activity to the same extent as the parent compound. A compound is regarded as an analogue within the context of the present application, if it exhibits the relevant activity to a degree of at least 1% (more preferably at least 5%, more preferably at least 10%, more preferably at least 20%, more preferably at least 30%, more preferably at least 40%, and more preferably at least 50%) of the activity of the parent compound. Thus, an "analogue of an amatoxin", as it is used herein, refers to a compound that is structurally related to any one of α-amanitin, β-amanitin, γ-amanitin, ε-amanitin, amanin, amaninamide, amanullin, and amanullinic acid as shown in Fig. 1 and that exhibits at least 1% (more preferably at least 5%, more preferably at least 10%, more preferably at least 20%, more preferably at least 30%, more preferably at least 40%, and more preferably at least 50%) of the inhibitory activity against mammalian RNA polymerase II as compared to at least one of α-amanitin, β-amanitin, γ-amanitin, ε-amanitin, amanin, amaninamide, amanullin, and amanullinic acid. An "analogue of an amatoxin" suitable for use in the present invention may even exhibit a greater inhibitory activity against mammalian RNA polymerase II than any one of α-amanitin, β-amanitin, γ-amanitin, ε-amanitin, amanin, amaninamide, amanullin, or amanullinic acid. The inhibitory activity might be measured by determining the concentration at which 50% inhibition occurs (IC₅₀ value). The inhibitory activity against mammalian RNA polymerase II can be determined indirectly by measuring the inhibitory activity on cell proliferation. A suitable assay for measuring inhibition of cell proliferation is described in the examples.

A "semisynthetic analogue" refers to an analogue that has been obtained by chemical synthesis using compounds from natural sources (e.g. plant materials, bacterial cultures, or cell cultures) as starting material. Typically, a "semisynthetic analogue" of the present invention has been synthesized starting from a compound isolated from a mushroom of the Amanita family. In contrast, a "synthetic analogue" refers to an analogue synthesized by so-called total synthesis from small (typically petrochemical) building blocks. Usually, this total synthesis is carried out without the aid of biological processes.

As used herein, an "aptamer conjugate" refers to a target-binding moiety toxin conjugate in which the target-binding moiety is a nucleic acid aptamer according to above alternative (iii).

A "linker" in the context of the present invention refers to a molecule that is connecting two components, each being attached to one end of the linker, and which increases the distance between two components and alleviates steric interference between these components, such as in the present case between the target-binding moiety and the amatoxin. In the absence of a linker, a direct linkage of amatoxin to the target-binding moiety may decrease the ability of the amatoxin to interact with RNA polymerase II. In particular embodiments, a linker has a continuous chains of between 1 and 30 atoms (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 atoms) in its backbone, i.e. the length of the linker is defined as the shortest connection as measured by the number of atoms or bonds between the amatoxin moiety and the target-binding moiety, wherein one side of the linker backbone has been reacted with the amatoxin and, the other side with a target-binding moiety. In the context of the present invention, a linker preferably is an C₁₋₂₀-alkylene, C₁₋₂₀-heteroalkylene, C₂₋₂₀-alkenylene, C₂₋₂₀-heteroalkenylene, C₂₋₂o-alkynylene, C₂₋₂₀-heteroalkynylene, cycloalkylene, heterocycloalkylene, arylene, heteroarylene, aralkylene, or a heteroaralkylene group, optionally substituted. The linker may contain one or more structural elements such as carboxamide, ester, ether, thioether, disulfide, urea, thiourea, hydrocarbon moieties and the like. The linker may also contain combinations of two or more of these structural elements. Each one of these structural elements may be present in the linker more than once, e.g. twice, three times, four times, five times, or six times. In some embodiments the linker may comprise a disulfide bond. It is understood that the linker has to be attached either in a single step or in two or more subsequent steps to the amatoxin and the target-binding moiety. To that end the linker to be will carry two groups, preferably at a proximal and distal end, which can (i) form a covalent bond to a group present in one of the components to be linked, preferably an activated group on an amatoxin or the target binding-peptide or (ii) which is or can be activated to form a covalent bond with a group on an amatoxin. Accordingly, it is preferred that chemical groups are at the distal and proximal end of the linker, which are the result of such a coupling reaction, e.g. an ester, an ether, a urethane, a peptide bond etc.

In the context of the present invention, the term "amatoxin" includes all cyclic peptides composed of 8 amino acids as isolated from the genus Amanita and described in Wieland, T. and Faulstich H. (Wieland T, Faulstich H., CRC Crit Rev Biochem. 1978 Dec;5(3):185-260), and furthermore includes all chemical derivatives thereof; further all semisynthetic analogues thereof; further all synthetic analogues thereof built from building blocks according to the master structure of the natural compounds (cyclic, 8 amino acids), further all synthetic or semisynthetic analogues containing non-hydroxylated amino acids instead of the hydroxylated amino acids, further all synthetic or semisynthetic analogues, in which the thioether sulfoxide moiety is replaced by a sulfide, sulfone, or by atoms different from sulfur, e.g. a carbon atom as in a carbaanalogue of amanitin, in each case wherein any such derivative or analogue is functionally active by inhibiting mammalian RNA polymerase II.

Functionally, amatoxins are defined as peptides or depsipeptides that inhibit mammalian RNA polymerase II. Preferred amatoxins are those with a functional group (e.g. a carboxylic group, an amino group, a hydroxy group, a thiol or a thiol-capturing group) that can be reacted with linker molecules or target-binding moieties as defined above. Amatoxins which are particularly suitable for the conjugates of the present invention are α-amanitin, β-amanitin, γ-amanitin, ε-amanitin, amanin, amaninamide, amanullin, and amanullinic acid as shown in Fig. 1 as well as salts, chemical derivatives, semisynthetic analogues, and synthetic analogues thereof. Particularly preferred amatoxins for use in the present invention are α-amanitin, β-amanitin, and amaninamide.

As used herein, a "chemical derivative" (or short: a "derivative") of a compound refers to a species having a chemical structure that is similar to the compound, yet containing at least one chemical group not present in the compound and/or deficient of at least one chemical group that is present in the compound. The compound to which the derivative is compared is known as the "parent" compound. Typically, a "derivative" may be produced from the parent compound in one or more chemical reaction steps.

In particular embodiments of the present invention, the amatoxin is selected from α-amanitin, β-amanitin, γ-amanitin, ε-amanitin, amanin, amaninamide, amanullin, or amanullinic acid, or from salts or analogues thereof.

In one aspect, the present invention relates to an amatoxin conjugate of Formula I 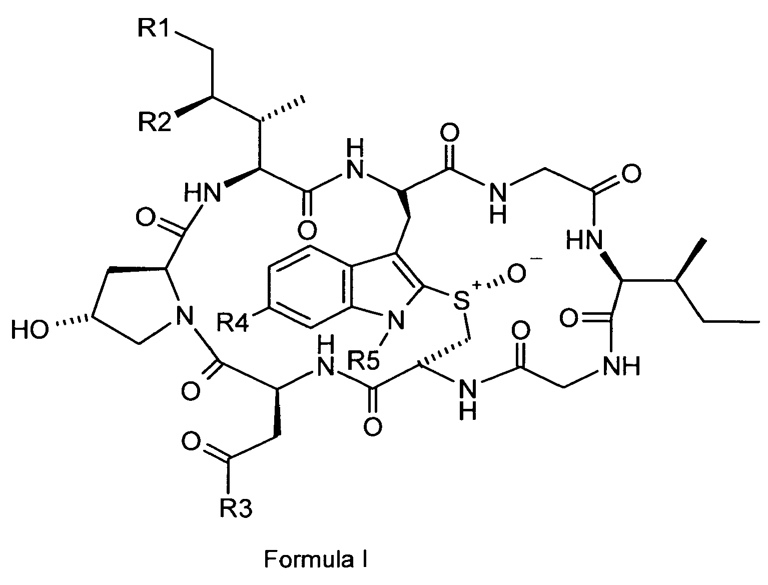 wherein:
- R1=: H or OH;
- R2=: H or OH;
- R3=: NH₂ or OH;
- R4=: OH or OC₁₋₆-alkyl; and
- R5=: L-T; wherein L is a linker; and T is a target binding moiety.

In a particular embodiment, the amatoxin conjugate of Formula I is a derivative of α-amanitin (R1 = OH; R2 = OH; and R3 = NH₂).

In a particular embodiment, R4 is O-Me.

In a particular embodiment, the linker is connected to the target-binding moiety via a urea moiety.

In the context of the present invention the term "connected to the target-binding moiety via a urea moiety" refers to a connection between the linker and the target-binding moiety, where the target-binding moiety is directly attached to the linker via an -NH-C(O)-NH- group.

In particular embodiments of the present invention, the target-binding moiety is connected to the linker L via an amino group present in the target-binding moiety, wherein said amino group forms part of said urea moiety.

In particular embodiments, the linker has a length of between 1 and 8 atoms, particularly between 1 and 6, more particularly between 1 and 4, and most particularly between 2 and 4 atoms.

In particular embodiments of the present invention, the linker L comprises one or more groups, particularly one, two or three groups, selected from the list of: alkylene, alkenylene, alkynylene, cycloalkylene, heteroalkylene, heteroalkenylene, heteroalkynylene, heterocycloalkylene, arylene, heteroarylene, aralkylene, and a heteroaralkylene group, wherein each group may optionally be independently substituted.

The term "alkylene" refers to a bivalent straight chain saturated hydrocarbon groups having from 1 to 20 carbon atoms, including groups having from 1 to 10 carbon atoms. In certain embodiments, alkylene groups may be lower alkylene groups. The term "lower alkylene" refers to alkylene groups having from 1 to 6 carbon atoms, and in certain embodiments from 1 to 5 or 1 to 4 carbon atoms. Examples of alkylene groups include, but are not limited to, methylene (-CH2-), ethylene (-CH2-CH2-), n-propylene, n-butylene, n-pentylene, and n-hexylene.

The term "alkenylene" refers to bivalent straight chain groups having 2 to 20 carbon atoms, wherein at least one of the carbon-carbon bonds is a double bond, while other bonds may be single bonds or further double bonds. The term "alkynylene" herein refers to groups having 2 to 20 carbon atoms, wherein at least one of the carbon-carbon bonds is a triple bond, while other bonds may be single, double or further triple bonds. Examples of alkenylene groups include ethenylene (-CH=CH-), 1-propenylene, 2-propenylene, 1-butenylene, 2-butenylene, 3-butenylene, and the like. Examples of alkynylene groups include ethynylene, 1-propynylene, 2-propynylene, and so forth.

As used herein, "cycloalkylene" is intended to refer to a bivalent ring being part of any stable monocyclic or polycyclic system, where such ring has between 3 and 12 carbon atoms, but no heteroatom, and where such ring is fully saturated, and the term "cycloalkenylene" is intended to refer to a bivalent ring being part of any stable monocyclic or polycyclic system, where such ring has between 3 and 12 carbon atoms, but no heteroatom, and where such ring is at least partially unsaturated (but excluding any arylene ring). Examples of cycloalkylenes include, but are not limited to, cyclopropylene, cyclobutylene, cyclopentylene, cyclohexylene, and cycloheptylene. Examples of cycloalkenylenes include, but are not limited to, cyclopentenylene and cyclohexenylene.

As used herein, the terms "heterocycloalkylene" and "heterocycloalkenylene" are intended to refer to a bivalent ring being part of any stable monocyclic or polycyclic ring system, where such ring has between 3 and about 12 atoms, and where such ring consists of carbon atoms and at least one heteroatom, particularly at least one heteroatom independently selected from the group consisting of N, O and S, with heterocycloalkylene referring to such a ring that is fully saturated, and heterocycloalkenylene referring to a ring that is at least partially unsaturated (but excluding any arylene or heteroarylene ring).

The term "arylene" is intended to mean a bivalent ring or ring system being part of any stable monocyclic or polycyclic system, where such ring or ring system has between 3 and 20 carbon atoms, but has no heteroatom, which ring or ring system consists of an aromatic moiety as defined by the "4n+2" π electron rule, including phenylene.

As used herein, the term "heteroarylene" refers to a bivalent ring or ring system being part of any stable mono- or polycyclic system, where such ring or ring system has between 3 and 20 atoms, which ring or ring system consists of an aromatic moiety as defined by the "4n+2" π electron rule and contains carbon atoms and one or more nitrogen, sulfur, and/or oxygen heteroatoms.

In the context of the present invention, the term "substituted" is intended to indicate that one or more hydrogens present in the backbone of a linker is replaced with a selection from the indicated group(s), provided that the indicated atom's normal valency, or that of the appropriate atom of the group that is substituted, is not exceeded, and that the substitution results in a stable compound. The term "optionally substituted" is intended to mean that the linker is either unsubstituted or substituted, as defined herein, with one or more substituents, as defined herein. When a substituent is a keto (or oxo, i.e. =O) group, a thio or imino group or the like, then two hydrogens on the linker backbone atom are replaced. Exemplary substituents include, for example, alkyl, alkenyl, alkynyl, cycloalkyl, heterocycloalkyl, aryl, heteroaryl, aralkyl, heteroaralkyl, acyl, aroyl, heteroaroyl, carboxyl, alkoxy, aryloxy, acyloxy, aroyloxy, heteroaroyloxy, alkoxycarbonyl, halogen, (thio)ester, cyano, phosphoryl, amino, imino, (thio)amido, sulfhydryl, alkylthio, acylthio, sulfonyl, a sulfate, a sulfonate, a sulfamoyl, a sulfonamido, nitro, azido, haloalkyl, inclucing perfluoroalkyl (such as trifluoromethyl), haloalkoxy, alkylsulfanyl, alkylsulfinyl, alkylsulfonyl, alkylsulfonylamino, arylsulfonoamino, phosphoryl, phosphate, phosphonate, phosphinate, alkylcarboxy, alkylcarboxyamide, oxo, hydroxy, mercapto, amino (optionally mono- or di-substituted, e.g. by alkyl, aryl, or heteroaryl), imino, carboxamide, carbamoyl (optionally mono- or di-substituted, e.g. by alkyl, aryl, or heteroaryl), amidino, aminosulfonyl, acylamino, aroylamino, (thio)ureido, arylthio)ureido, alkyl(thio)ureido, cycloalkyl(thio)ureido, aryloxy, aralkoxy, or -O(CH2)n-OH, -O(CH2)n-NH2, -O(CH2)nCOOH, -(CH2)nCOOH, -C(O)O(CH2)nR, -(CH2)nN(H)C(O)OR, or - N(R)S(O)2R wherein n is 1-4 and R is independently selected from hydrogen, -alkyl, - alkenyl, -alkynyl, -cycloalkyl, -cycloalkenyl, -(C-linked-heterocycloalkyl), -(C-linked-heterocycloalkenyl), -aryl, and -heteroaryl, with multiple degrees of substitution being allowed. It will be understood by those skilled in the art that substituents, such as heterocycloalkyl, aryl, heteroaryl, alkyl, etc., or functional groups such as -OH, -NHR etc., can themselves be substituted, if appropriate. It will also be understood by those skilled in the art that the substituted moieties themselves can be substituted as well when appropriate.

In particular embodiments of the present invention, wherein the linker L comprises m groups selected from the list of: alkylene, alkenylene, alkynylene, cycloalkylene, heteroalkylene, heteroalkenylene, heteroalkynylene, heterocycloalkylene, arylene, heteroarylene, aralkylene, and a heteroaralkylene group, wherein each group may optionally be independently substituted, the linker further comprises n moiety independently selected from one of the following moieties: a disulfide (-S-S-), an ether (-O-), a thioether (-S-), an amine (-NH-), an ester (-O-C(=O)- or -C(=O)-O-), a carboxamide (-NH-C(=O)- or -C(=O)-NH-), a urethane (-NH-C(=O)-O- or -O-C(=O)-NH-), and a urea moiety (-NH-C(=O)-NH-), wherein m = n+1. In particular embodiments, m is 2 and n is 1, or m is 3 and n is 2. In particular embodiments, the linker comprises 2 or 3 unsubstituted alkylene groups, and 1 or 2, respectively, disulfide, ether, thioether, amine, ester, carboxamide, urethane or urea moieties linking the unsubstituted alkylene groups.

In particular embodiments of the present invention the target-binding moiety specifically binds to an epitope that is present on a tumour cell.

In particular embodiments of the present invention the target-binding moiety specifically binds to an epitope of Her-2/neu or epithelial cell adhesion molecule (EpCAM).

In particular embodiments of the present invention the target-binding moiety is selected from the group consisting of: antibody or antigen-binding fragment thereof; antibody-like protein; and nucleic acid aptamer.

In particular embodiments of the present invention the antibody or the antigen-binding fragment thereof is selected from a diabody, a tetrabody, a nanobody, a chimeric antibody, a deimmunized antibody, a humanized antibody or a human antibody.

In particular embodiments of the present invention the antigen binding fragment is selected from the group consisting of Fab, F(ab')₂, Fd, Fv, single-chain Fv, and disulfide-linked Fvs (dsFv).

In particular embodiments, the antibody is herceptin or HEA125, or an antibody fragment comprising the antigen binding fragment of herceptin or HEA125.

In particular embodiments, more than one amatoxin molecule is coupled to one target-binding moiety. An increase of the number of amatoxins per conjugate will also increase the toxicity. Accordingly, in a particular embodiment the ratio of target-binding moiety to amatoxin is between 1 target-binding moiety to between 2 and 15 amatoxin molecules, particularly 2, 3, 4, 5, 6, 7, 8, 9, 10, 11, 12, 13, 14, or 15. For the purpose of the calculation of the ratio in case of antibody dimers such as IgGs, the dimer is considered as one target-binding moiety.

In particular embodiments of the present invention, the conjugate is for use as a medicament.

In particular embodiments of the present invention, the conjugate is for use in the treatment of cancer in a patient, wherein the cancer is selected from the group consisting of pancreatic cancer, cholangiocarcinoma, breast cancer, colorectal cancer, lung cancer, prostate cancer, ovarian cancer, stomach cancer, kidney cancer, malignant melanoma, leukemia, and malignant lymphoma.

In yet another aspect, the present invention relates to a method for the treatment of cancer in a patient, wherein the cancer is selected from the group consisting of pancreatic cancer, cholangiocarcinoma, breast cancer, colorectal cancer, lung cancer, prostate cancer, ovarian cancer, stomach cancer, kidney cancer, malignant melanoma, leukemia, and malignant lymphoma, comprising the step of administering a conjugate of the present invention to a patient in need thereof.

As used herein, a "patient" means any mammal or bird who may benefit from a treatment with the target-binding moiety toxin conjugates described herein. Preferably, a "patient" is selected from the group consisting of laboratory animals (e.g. mouse or rat), domestic animals (including e.g. guinea pig, rabbit, chicken, pig, sheep, goat, camel, cow, horse, donkey, cat, or dog), or primates including human beings. It is particularly preferred that the "patient" is a human being.

As used herein, "treat", "treating" or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, the treatment may comprise administering a conjugate or a pharmaceutical composition according to the present invention to a patient, wherein "administering" includes in vivo administration, as well as administration directly to tissue ex vivo, such as vein grafts.

In particular embodiments, a therapeutically effective amount of the conjugate of the present invention is used.

A "therapeutically effective amount" is an amount of a therapeutic agent sufficient to achieve the intended purpose. The effective amount of a given therapeutic agent will vary with factors such as the nature of the agent, the route of administration, the size and species of the animal to receive the therapeutic agent, and the purpose of the administration. The effective amount in each individual case may be determined empirically by a skilled artisan according to established methods in the art.

In a further aspect, the present invention relates to a pharmaceutical composition comprising the conjugate according to the present invention, further comprising one or more pharmaceutically acceptable diluents, carriers, excipients, fillers, binders, lubricants, glidants, disintegrants, adsorbents; and/or preservatives.

"Pharmaceutically acceptable" means approved by a regulatory agency of the Federal or a state government or listed in the U.S. Pharmacopeia or other generally recognized pharmacopeia for use in animals, and more particularly in humans.

In particular embodiments, the pharmaceutical composition is used in the form of a systemically administered medicament. This includes parenterals, which comprise among others injectables and infusions. Injectables are formulated either in the form of ampoules or as so called ready-for-use injectables, e.g. ready-to-use syringes or single-use syringes and aside from this in puncturable flasks for multiple withdrawal. The administration of injectables can be in the form of subcutaneous (s.c.), intramuscular (i.m.), intravenous (i.v.) or intracutaneous (i.c.) application. In particular, it is possible to produce the respectively suitable injection formulations as a suspension of crystals, solutions, nanoparticular or a colloid dispersed systems like, e.g. hydrosols.

Injectable formulations can further be produced as concentrates, which can be dissolved or dispersed with aqueous isotonic diluents. The infusion can also be prepared in form of isotonic solutions, fatty emulsions, liposomal formulations and microemulsions. Similar to injectables, infusion formulations can also be prepared in the form of concentrates for dilution. Injectable formulations can also be applied in the form of permanent infusions both in in-patient and ambulant therapy, e.g. by way of mini-pumps.

It is possible to add to parenteral drug formulations, for example, albumin, plasma, expander, surface-active substances, organic diluents, pH-influencing substances, complexing substances or polymeric substances, in particular as substances to influence the adsorption of the target-binding moiety toxin conjugates of the invention to proteins or polymers or they can also be added with the aim to reduce the adsorption of the target-binding moiety toxin conjugates of the invention to materials like injection instruments or packaging-materials, for example, plastic or glass.

The target-binding moiety toxin conjugates of the invention can be bound to microcarriers or nanoparticles in parenterals like, for example, to finely dispersed particles based on poly(meth)acrylates, polylactates, polyglycolates, polyamino acids or polyether urethanes. Parenteral formulations can also be modified as depot preparations, e.g. based on the "multiple unit principle", if the target-binding moiety toxin conjugates of the invention are introduced in finely dispersed, dispersed and suspended form, respectively, or as a suspension of crystals in the medicament or based on the "single unit principle" if the target-binding moiety toxin conjugate of the invention is enclosed in a formulation, e.g. in a tablet or a rod which is subsequently implanted. These implants or depot medicaments in single unit and multiple unit formulations often consist of so called biodegradable polymers like e.g. polyesters of lactic acid and glycolic acid, polyether urethanes, polyamino acids, poly(meth)acrylates or polysaccharides.

Adjuvants and carriers added during the production of the pharmaceutical compositions of the present invention formulated as parenterals are preferably aqua sterilisata (sterilized water), pH value influencing substances like, e.g. organic or inorganic acids or bases as well as salts thereof, buffering substances for adjusting pH values, substances for isotonization like e.g. sodium chloride, sodium hydrogen carbonate, glucose and fructose, tensides and surfactants, respectively, and emulsifiers like, e.g. partial esters of fatty acids of polyoxyethylene sorbitans (for example, Tween®) or, e.g. fatty acid esters of polyoxyethylenes (for example, Cremophor®), fatty oils like, e.g. peanut oil, soybean oil or castor oil, synthetic esters of fatty acids like, e.g. ethyl oleate, isopropyl myristate and neutral oil (for example, Miglyol®) as well as polymeric adjuvants like, e.g. gelatine, dextran, polyvinylpyrrolidone, additives which increase the solubility of organic solvents like, e.g. propylene glycol, ethanol, N,N-dimethylacetamide, propylene glycol or complex forming substances like, e.g. citrate and urea, preservatives like, e.g. benzoic acid hydroxypropyl ester and methyl ester, benzyl alcohol, antioxidants like e.g. sodium sulfite and stabilizers like e.g. EDTA.

When formulating the pharmaceutical compositions of the present invention as suspensions in a preferred embodiment thickening agents to prevent the setting of the target-binding moiety toxin conjugates of the invention or, tensides and polyelectrolytes to assure the resuspendability of sediments and/or complex forming agents like, for example, EDTA are added. It is also possible to achieve complexes of the active ingredient with various polymers. Examples of such polymers are polyethylene glycol, polystyrol, carboxymethyl cellulose, Pluronics® or polyethylene glycol sorbit fatty acid ester. The target-binding moiety toxin conjugates of the invention can also be incorporated in liquid formulations in the form of inclusion compounds e.g. with cyclodextrins. In particular embodiments dispersing agents can be added as further adjuvants. For the production of lyophilisates scaffolding agents like mannite, dextran, saccharose, human albumin, lactose, PVP or varieties of gelatine can be used.

In a further aspect the present invention is directed to a method of treating pancreatic cancer, cholangiocarcinoma, breast cancer, colorectal cancer, lung cancer, prostate cancer, ovarian cancer, stomach cancer, kidney cancer, malignant melanoma, leukemia, or malignant lymphoma in a patient in need thereof, comprising administering to the patient an effective amount of a conjugate or pharmaceutical composition of the present invention.

In another aspect, the present invention relates to an amatoxin-conjugation molecule comprising a leaving group X attached to a linker L connected to an amatoxin, or an analogue of an amatoxin, wherein the linker L is connected to the amatoxin via the 1'-N atom of amino acid 4.

In certain embodiments of such aspect, the present invention relates to an amatoxin-conjugation molecule of Formula I 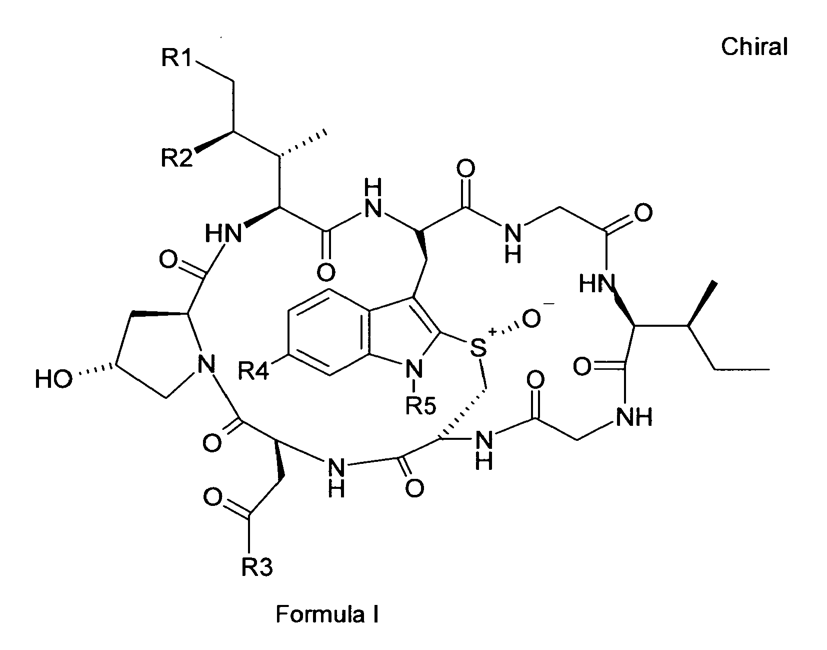 wherein:
- R1=: H or OH;
- R2=: H or OH;
- R3=: NH₂ or OH;
- R4=: OH or OC₁₋₆-alkyl; and
- R5=: L-X; wherein L is a linker; and X is a leaving group that can be replaced by a nucleophilic group of a target-binding moiety.

In a particular embodiment, the nucleophilic group is a primary amine.

In a particular embodiment, the amatoxin-conjugation molecule of Formula I is a derivative of α-amanitin (R1 = OH; R2 = OH; and R3 = NH₂).

In a particular embodiment, R4 is O-Me.

In particular embodiments, the linker has a length of between 1 and 8 atoms, particularly between 1 and 6, more particularly between 1 and 4, and most particularly between 2 and 4 atoms.

In certain embodiments, the linker L is an alkylene, heteroalkylene, alkenylene, heteroalkenylene, alkynylene, heteroalkynylene, cycloalkylene, heterocycloalkylene, arylene, heteroarylene, aralkylene, or a heteroaralkylene group, optionally substituted.

In certain embodiments, the linker L comprises a moiety selected from one of the following moieties: a disulfide, an ether, an amine, an ester, a carboxamide, a urethane, and a urea moiety.

In certain embodiments, the functional group X is selected from: ^{t}butyloxy, succinimidyloxy, 1-O-succinimidyloxy-3-sulfonate (Sulfo-NHS), O-(4-nitrophenyloxy), O-(3-nitrophenyloxy), O-(2,4-dinitrophenyloxy), O-(2,4-dichloro-6-nitrophenyloxy), pentafluorophenyloxy, pentachlorophenyloxy, O-(2,4,5-trichlorophenyloxy), O-(3,4-dihydro-3-hydroxy-4-oxo-1,2,3-benzotriazine-3-yl), O-(endo-1-hydroxy-5-norbornene-2,3-dicarboximide-1-yl), 1-phthalimidoyloxy, 1-benzotriazolyloxy, 1-(7-aza-benzotriazolyl)oxy, and N-imidazolyl.

In yet another aspect, the present invention relates to a method for synthesizing an amatoxin conjugate of the present invention, comprising the step of reacting an amatoxin-conjugation molecule of the present invention with a target-binding moiety comprising a nucleophilic group, particularly a primary amine.

In yet another aspect, the present invention relates to a method for synthesizing an amatoxin-conjugation molecule of the present invention, comprising the step of (i) reacting an amatoxin with a linker molecule Y-L-X, wherein Y is a moiety that can react with 1'-N atom of amino acid 4 of the amatoxin, and wherein X is a is a leaving group that can be replaced by a nucleophilic group of a target-binding moiety, particularly by a primary amine.

In yet another aspect, the present invention relates to a method for synthesizing an amatoxin-conjugation molecule of the present invention, comprising the step of (i) reacting an amatoxin with a linker molecule Y-L-Z, wherein Y is a moiety that can react with 1'-N atom of amino acid 4 of the amatoxin, and wherein Z is a moiety, which can be converted into a leaving group X, that can be replaced by a nucleophilic group of a target-binding moiety, particularly by a primary amine.

In certain embodiments, the amatoxin is reacted with a linker molecule Y-L-X, or Y-L-Z, respectively, wherein Y is selected from the group consisting of: O-Tos (O-Tos = tosylate); I; Br, and O-Tf (O-Tf = triflate). In certain such embodiments, the amatoxin is treated with Y-L-X, or Y-L-Z, respectively, in the presence of a base, particularly KO^{t}Bu, LiOH or NaH, in a solvent, particularly in a polar, aprotic solvent such as DMF, DMSO, or NMP.

In certain embodiments, the group Z is a protected primary amino group, particularly NH-C(=O)OtBu. In particular such embodiments, the protected primary amino is first deprotected and then activated by a carbonic acid derivative, such as dihydroxysuccinimido carbonate (DSC).

In another aspect, the present invention relates to an amatoxin-linker molecule of Formula I, wherein R1 to R4 are as defined above; and wherein R5 = L-Z; wherein L is a linker; and wherein Z is a moiety, which can be converted into a leaving group X, that can be replaced by a nucleophilic group of a target-binding moiety, particularly by a primary amine.

### EXAMPLES

In the following, the invention is explained in more detail by non-limiting examples:

### Example 1

### Synthesis of N1' Linker Amanitines

### 1.1 Preparation of N1'-6-aminohexyl-6'-O-methyl-α-amanitin (HDP 30.0378)

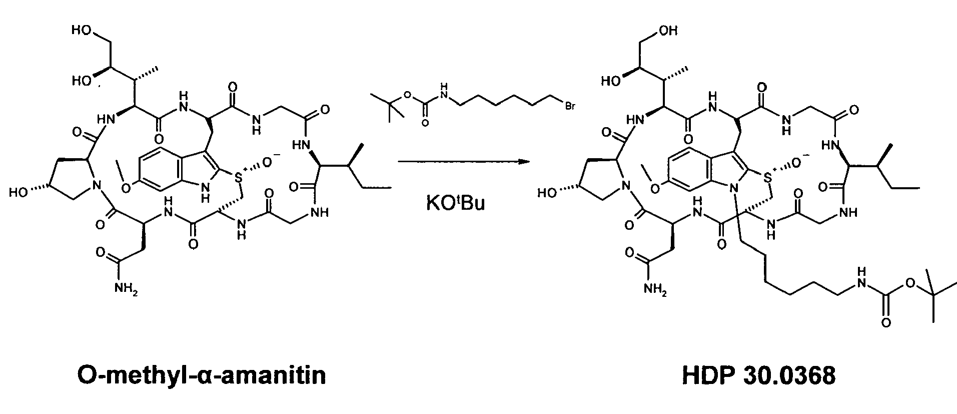

4.86 mg (5.21 µmol) O-methyl-α-amanitin were dissolved in 500 µl dry DMSO. Under argon, 11.68 mg (41.67 µmol) Boc-aminohexylbromide and 100 µl of a 52.1 mM solution of potassium-t-butanolate in DMSO were added. Additional potassium-t-butanolate portions of the 5.21 mM stock solution were added: 50 µl at 1.5 h; 50 µl at 4 h, and 50 µl at 6 h. After 19 h, 100 µl potassium-t-butanolate and 11.96 mg (41.67 µmol) Boc-aminohexylbromide were added. After 21 h, the reaction mixture was quenched with 104 µl of a 100 mM acetic acid solution in DMSO. The crude product was purified on a LaPrep-HPLC: column: Kromasil 100-C18, 10 µm, 250 x 20 mm, with methanol/water ( 0.05% TFA), flow: 26.0 ml/min, detection at λ = 295 nm. Solvent A: 95% water: 5% methanol, 0.05% trifluoroacetic acid. Solvent B: 10% water: 90% methanol, 0.05% trifluoroacetic acid. Gradient: 0-5 min 100% A; 5-20 min 0% A; 20-25 min 0% A; 25-27 min 100% A; 27-35 min 100% A; the fraction with the retention time of 19.2 - 20.9 min was collected and the solvents evaporated to a solid.

4.81 mg (81%) **HDP 30.0368**; MS: 1133 (M+H⁺).

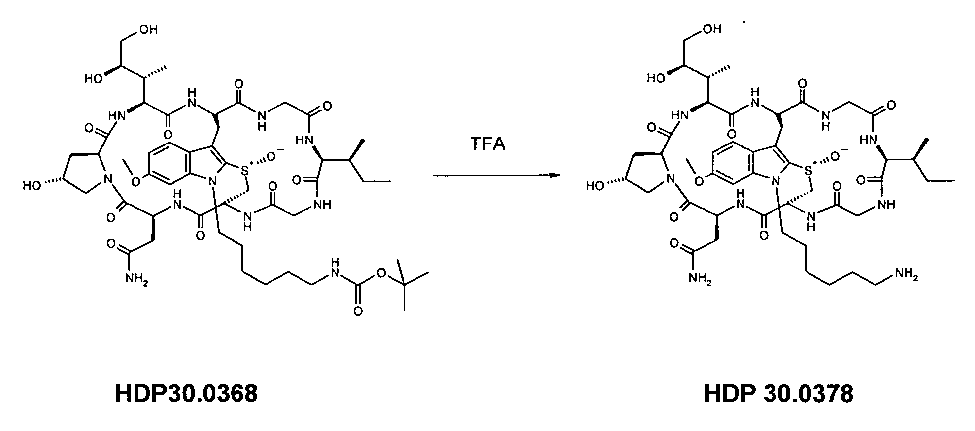

4.75 mg (4.19 µmol) HDP 30.0368 were dissolved in 200 µl trifluoroacetic acid and stirred for 2 min. The reaction mixture was evaporated to dryness at ambient temperature and co-evaporated with 1000 µl toluene and 10000 µl acetonitrile. The crude product was purified on a LaPrep-HPLC: column: Kromasil 100-C18, 10 µm, 250 x 20 mm, with methanol/water (0.05% TFA), flow: 26.0 ml/min, detection at λ = 295 nm. Solvent A: 95% water: 5% methanol, 0.05% trifluoroacetic acid. Solvent B: 10% water: 90% methanol, 0.05% trifluoroacetic acid. Gradient: 0-5 min 100% A; 5-20 min 0% A; 20-25 min 0% A; 25-27 min 100% A; 27-35 min 100% A; the fraction with the retention time of 14.9-15.5 min was collected and the solvents evaporated and freeze dried to a powder.

1.54mg (32%) **HDP 30.0378**; MS: 1033 (M+H+).

### 1.2 Preparation of N1'-8-aminooctyl-6'-O-methyl-α-amanitin (HDP 30.0516)

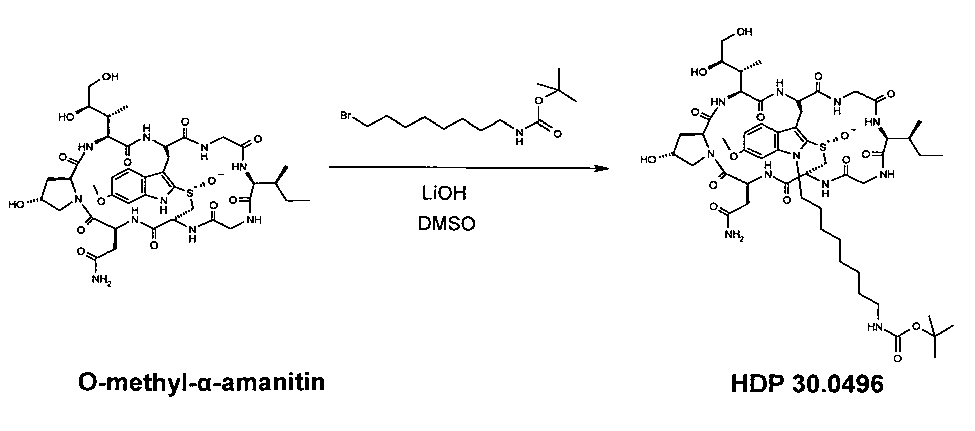

12.43 mg (13.32 µmol) O-methyl-α-methylamanitin were dissolved in 750 µl dry DMSO. 40.00 mg (129.76 µmol) N-Boc-8-aminobromoactane and 120 µl of a 0.1 M LiOH solution in DMSO/water 1:1 were added. The reaction mixture was stirred at room temperature under argon. After 18 h additional 80 µl LiOH were added followed by 40 µl 36 h, 50 µl 39 h and 80 µl 44 h later. The DMSO/water was removed in high vacuum and the residue was purified on a LaPrep-HPLC: column: Kromasil 100-C18, 10 µm, 250 x 20 mm, with methanol/water (0.05% TFA), flow: 10.0 ml/min, detection at λ = 230nm. Solvent A: 95% water: 5% methanol, 0.05% trifluoroacetic acid. Solvent B: 10% water: 90% methanol, 0.05% trifluoroacetic acid. Gradient: 0-5 min 80% A; 5-10 min 50% A; 10-20 min 30% A; 20-50 min 20% A; the fraction with the retention time of 37.0 min was collected and the solvents evaporated in vacuum.

3.96 mg (26%) **HDP 30.0496** as a white powder. MS: 1160 (M+H⁺).

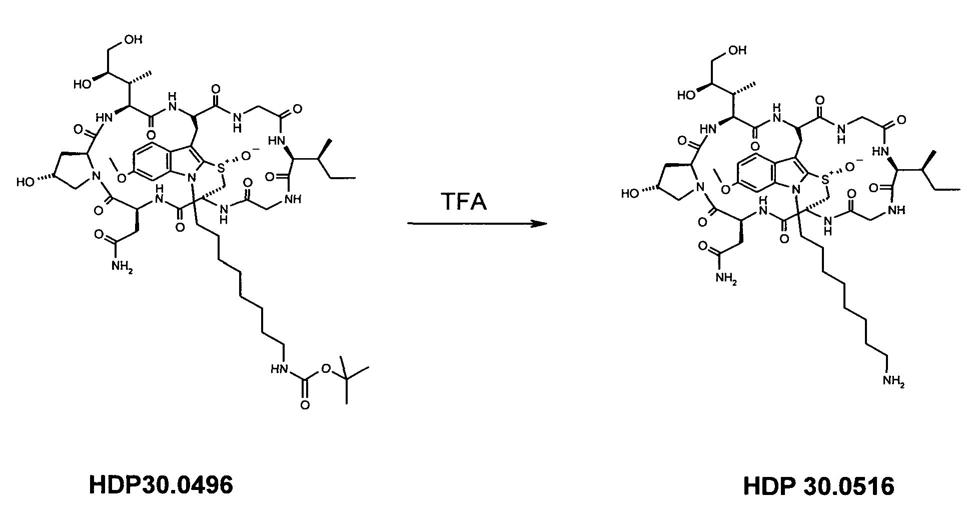

3.96 mg (3.41 µmol) HDP 30.0496 were dissolved in 300 µl trifluoroacetic acid and stirred for 2.5 min at room temperature. The reaction mixture was diluted with 1000 µl toluene and evaporated to dryness at ambient temperature. The residue was co-evaporated two times with the same volume of toluene. The residue was purified on a LaPrep-HPLC: column: Kromasil 100-C18, 10 µm, 250 x 20 mm, with methanol/water (0.05% TFA), flow: 10.0 ml/min, detection at λ = 230 nm. Solvent A: 95% water: 5% methanol, 0.05% trifluoroacetic acid. Solvent B: 10% water: 90% methanol, 0.05% trifluoroacetic acid. Gradient: 0-5 min 80% A; 5-10 min 50% A; 10-20 min 30% A; 20-50 min 20% A; the fraction with the retention time of 21.0 min was collected and the solvents evaporated in vacuum.

2.66mg (74%) **HDP 30.0516** as a white powder. MS: 1060 M+H; 1083 (M+Na⁺).

### Example 2

### Synthesis of Amatoxin Conjugate

### Synthesis of HDP 30.0378 conjugate Her-DSC-30.0378 [2.8]

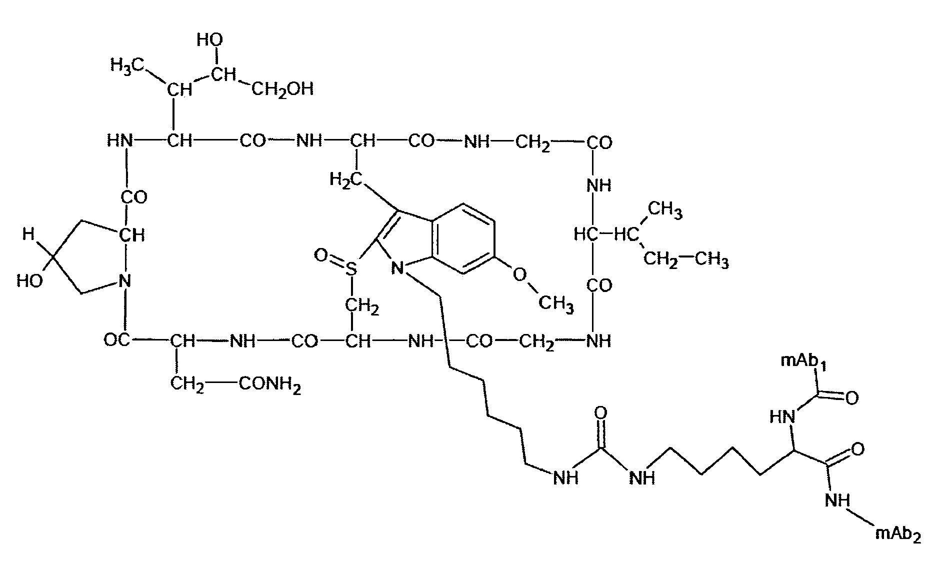

1.0 mg HDP 30.0378 was dissolved in 100 µl dry dimethylformamide (DMF). Under argon and stirring at room temperature 10.4 µl of a solution of dihydroxysuccinimido carbonate (DSC) in DMF (2.56 mg in 100 µl DMF) and 2.1 µl triethylamine were added at once. The reaction mixture was stirred at room temperature. After 12 h, 30 ml cold diethylether were added. The precipitate was collected and washed several times with diethylether and dried in vacuum. The remaining solid was taken up in 200 µl DMF = solution A. 12.0 mg Herceptin were dissolved in 4.0 ml phosphate buffered saline (PBS, pH = 7.4) = solution B. Solution A and solution B were combined. The Herceptin amanitin-linker solution was shaken at 4°C for 14 h and separated by Sephadex G-25 gelfiltration chromatography (XK-16 column; 2 ml/min). The G-25 column was pre-washed with 500 ml PBS solutions, pH = 7.4. The Her-DSC-30.0378 conjugate fraction was detected by UV absorption. Protein concentration was determined by RotiQuant-Assay (Carl Roth; Germany). Amanitin payload of Herceptin was determined by determination of UV absorption at A = 280 nm and A = 310 nm. A toxin payload of 2.8 amanitin molecules for each Herceptin molecule was calculated.

### Example 3

### Cytotoxicity of Her-DSC-30.0378 [2.8] on a HER2-positive tumor cell line in vitro

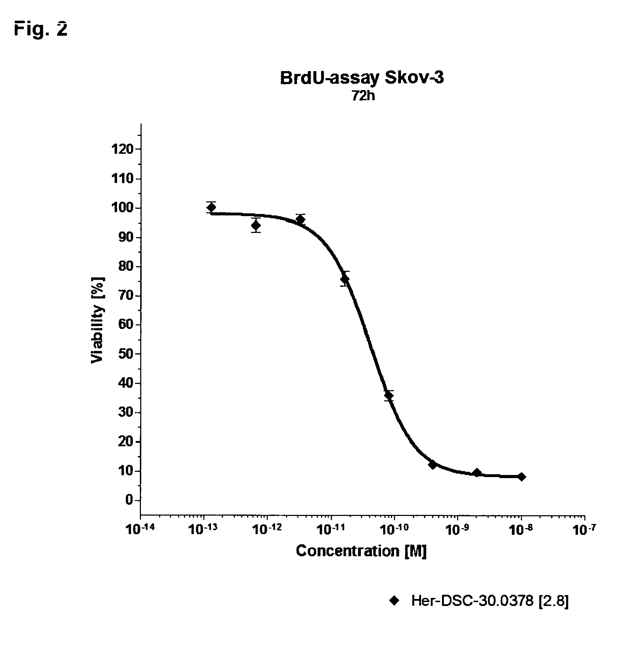

Cytotoxic activity of Her-DSC-30.0378 [2.8] was evaluated with the HER2-positive tumor cell line SK-OV-3 (ovar) and a chemiluminescent BrdU incorporation assay (Roche Diagnostics) *in vitro.* Cell viability was determined after 72 h incubation with different concentrations of Her-DSC-30.0378 [2.8] at 37°C and 5% CO2 by measurement of fixed and permealized cells with an anti-BrdU-HRP antibody in a BMG Labtech Optima microplate reader. EC50 value of dose-response curve was calculated by Graphpad Prism 4.0 software. The EC50 for Her-DSC-30.0378 [2.8] with SK-OV-3 cells was 4.1x10-11 M. (see Figure 2).

## Claims

1. An amatoxin conjugate of Formula I 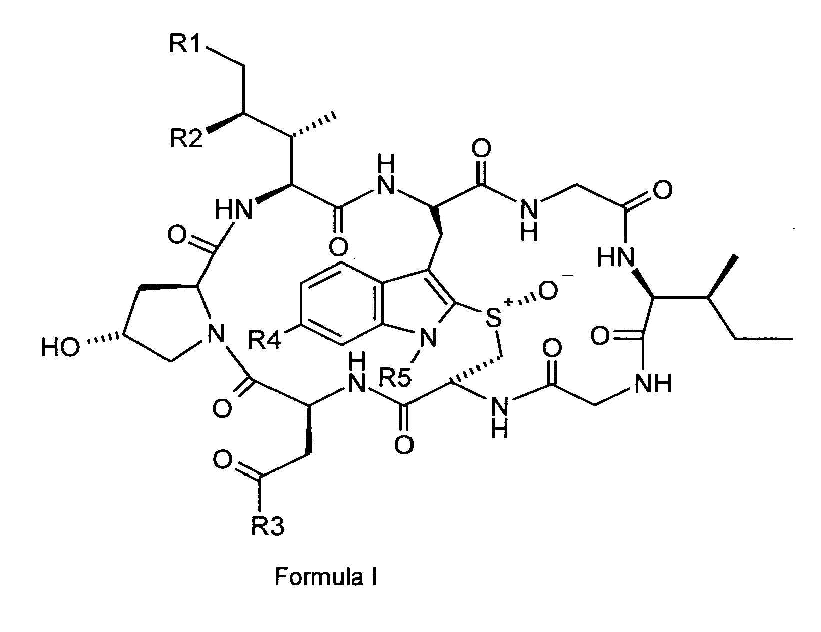 wherein:
R1= H or OH;
R2= H or OH;
R3= NH₂ or OH;
R4= OH or OC₁₋₆-alkyl; and
R5= L-T; wherein L is a linker; and T is a target binding moiety.

2. The conjugate of claim 1, wherein the linker is connected to the target-binding moiety via a urea moiety.

3. The conjugate of claim 1 or 2, wherein the target-binding moiety is connected to the linker L via an amino group present in the target-binding moiety, wherein said amino group forms part of said urea moiety.

4. The conjugate of any one of claims 1 to 3, wherein the linker has a length of between 1 and 8 atoms, particularly between 1 and 6, more particularly between 1 and 4, and most particularly between 2 and 4 atoms.

5. The conjugate of any one of claims 1 to 4, wherein the linker L is an alkylene, heteroalkylene, alkenylene, heteroalkenylene, alkynylene, heteroalkynylene, cycloalkylene, heterocycloalkylene, arylene, heteroarylene, aralkylene, or a heteroaralkylene group, optionally substituted.

6. The conjugate of any one of claims 1 to 5, wherein the linker L comprises a moiety selected from one of the following moieties: a disulfide, an ether, an amine, an ester, a carboxamide, a urethane, and a urea moiety.

7. The conjugate of any one of claims 1 to 6 wherein the target-binding moiety specifically binds to an epitope that is present on a tumour cell, particularly wherein the target-binding moiety specifically binds to an epitope of epithelial cell adhesion molecule (EpCAM).

8. The conjugate of any one of claims 1 to 7, wherein the target-binding moiety is selected from the group consisting of:
(i) antibody or antigen-binding fragment thereof;
(ii) antibody-like protein; and
(iii) nucleic acid aptamer.

9. The conjugate of claim 8, wherein the antibody or the antigen-binding fragment thereof is selected from a diabody, a tetrabody, a nanobody, a chimeric antibody, a deimmunized antibody, a humanized antibody or a human antibody.

10. The target-binding moiety toxin conjugate of claim 8 or 9, wherein the antigen binding fragment is selected from the group consisting of Fab, F(ab')₂, Fd, Fv, single-chain Fv, and disulfide-linked Fvs (dsFv).

11. The conjugate of any one of claims 1 to 10 for use as a medicament.

12. The conjugate of any one of claims 1 to 11 for use in the treatment of cancer in a patient, wherein the cancer is selected from the group consisting of pancreatic cancer, cholangiocarcinoma, breast cancer, colorectal cancer, lung cancer, prostate cancer, ovarian cancer, stomach cancer, kidney cancer, malignant melanoma, leukemia, and malignant lymphoma.

13. Pharmaceutical composition comprising the conjugate according to any one of claims 1 to 10 and further comprising one or more pharmaceutically acceptable diluents, carriers, excipients, fillers, binders, lubricants, glidants, disintegrants, adsorbents; and/or preservatives.

14. An amatoxin-conjugation molecule of Formula I, wherein R1 to R4 are as defined in claim 1; and wherein R5 = L-X; wherein L is a linker; and X is a leaving group that can be replaced by a nucleophilic group of a target-binding moiety, particularly by a primary amine.

15. The amatoxin-conjugation molecule claim 14, wherein X is selected from: -^{t}butyloxy, -succinimidyloxy, -1-O-succinimidyloxy-3-sulfonate (-Sulfo-NHS), -O-(4-nitrophenyloxy), -O-(3-nitrophenyloxy), -O-(2,4-dinitrophenyloxy), -O-(2,4-dichloro-6-nitrophenyloxy), -pentafluorophenyloxy, -pentachlorophenyloxy, -O-(2,4,5-trichlorophenyloxy), -O-(3,4-dihydro-3-hydroxy-4-oxo-1,2,3-benzotriazine-3-yl), -O-(endo-1-hydroxy-5-norbornene-2,3-dicarboximide-1-yl), -1-phthalimidoyloxy, -1-benzotriazolyloxy, -1-(7-aza-benzotriazolyl)oxy, ), and -N-imidazolyl.

16. A method for synthesizing a conjugate of any one of claims 1 to 12, comprising the step of reacting an amatoxin-conjugation molecule of claim 14 or 15 with a target-binding moiety comprising a nucleophilic group, particularly a primary amine.
